# FASCICULE DE BREVET EUROPEEN

(11) **EP 2 280 660 B1**
(45) Date de publication et mention de la délivrance du brevet: **01.01.2014**
(21) Numéro de dépôt: 09765993.2
(22) Date de dépôt: 20.05.2009
(51) Int. Cl.: A61B 17/70

(54) **DISPOSITIF DE STABILISATION POSTERIEUR DYNAMIQUE EPOUSANT LA LORDOSE ANATOMIQUE**
VORRICHTUNG ZUR DYNAMISCHEN NACHTRÄGLICHEN STABILISIERUNG ZUR ANPASSUNG DER ANATOMISCHEN LORDOSE
DEVICE FOR DYNAMIC POSTERIOR STABILISATION MATCHING THE ANATOMIC LORDOSIS

(30) Priorité: 23.05.2008 FR 0802807
(43) Date de publication de la demande: 09.02.2011
(73) Titulaire: Biospine Implants, 33600 Pessac (FR)
(72) Inventeur: FORTIN, Frédéric, F-33600 Pessac (FR); ROBIN, Johann, F-33130 Bègles (FR); GILLES, Olivier, F-33600 Pessac (FR); SENNEQUIER, Brice, F-33600 Pessac (FR)
(86) Numéro de dépôt international: PCT/FR2009/000584
(87) Numéro de publication internationale: WO 2009/153431

(56) Documents cités:
- WO-A-03/007828
- FR-A- 2 869 524
- JP-A- 2002 224 131

## Description

### DOMAINE DE L'INVENTION

L'invention concerne un dispositif de stabilisation vertébrale postérieure dynamique épousant la lordose anatomique, fixé entre deux vis pédiculaires qui amortit toutes les sollicitations mécaniques et qui permet une mobilité contrôlée du segment intervertébral.

Ce dispositif va présenter de nouveaux moyens avec des avantages fonctionnels, supérieurs à ceux de l'art antérieur.

### ART ANTERIEUR :

On connaît de nombreux systèmes de fixations vertébrales postérieures rigidifiant un certain nombre de vertèbres en les privant de toute mobilité pour permettre ainsi d'encaisser toutes contraintes mécaniques: Cependant, la première vertèbre adjacente à ce bloc rigide garde toute sa mobilité et cette discontinuité brutale entre le bloc rigide et cette vertèbre libre engendre très souvent une hyper sollicitation des éléments de liaison. Il s'en suit une accélération de la dégénérescence de ce niveau.

Ce problème n'a été que très partiellement résolu par des systèmes semi-rigides conçus pour créer une rigidité intermédiaire entre les vertèbres mobiles et les vertèbres fixes. Ces systèmes présentant les inconvénients suivants :
- soit ils travaillent uniquement en traction. C'est le cas de tous les dispositifs basés sur les ligaments artificiels. Ces systèmes sont peu élastiques et laissent à l'appréciation de l'opérateur le soin de régler la tension rendant ainsi aléatoire les caractéristiques mécaniques en particulier dans le mode de fonctionnement traction compression qui nous concerne le plus souvent.
- soit ils travaillent en compression avec une butée en traction, ce qui rend ces dispositifs inefficaces dés qu'ils doivent assister des déplacements en extension.

Quoiqu'il en soit aucun des dispositifs connus ne résout entièrement le problème posé, à savoir : amortir les sollicitations mécaniques existant en traction compression et en flexion auxquelles deux vertèbres peuvent être soumises, en respectant la lordose anatomique, et en autorisant des débattements compatibles avec les sollicitations du corps humain.

Nous citerons comme première antériorité le brevet FR 2771280, Dispositif de liaison vertébral résilient composés de deux tiges mobiles raccordées par l'intermédiaire d'un élément amortisseur fait de matériaux métalliques d'élasticité modérée et ayant une rigidité importante. Ce dispositif ne peut en aucun cas avoir les mêmes fonctions et la même souplesse que celles obtenues par la présente invention. Ses performances sont limitées et la mobilité du dispositif est plus proche de celle d'une tige métallique rigide que de la mobilité que va procurer la présente invention.

Nous citerons comme deuxième antériorité le document n° EP 0669109 « Stabilisierung von benachbarten Rückenwirbeln »

Cette antériorité est un cylindre creux en polyuréthane comprimé entre deux têtes de vis pédiculaires réalisé à partir d'un ligament tendu qui traverse le cylindre creux et qui est fixé aux vis par serrage des bouchons. Outre qu'il n'y a pas de possibilité d'obliquité du cylindre en polyuréthane qui ne respecte donc pas la lordose anatomique, ce dernier est très raide et autorise très peu de mobilité du segment intervertébral. Notre invention s'attache au contraire à faciliter la mobilité du segment intervertébral traité en le faisant tendre vers une position anatomique normale (respect de l'angle de lordose lombaire).

Nous citerons une troisième antériorité plus récente soit la demande PCT WO03007828 (le préambule de la revendication 1 est basé sur ce document) qui décrit et revendique « un dispositif de liaison vertébral souple ».

Cette antériorité, bien qu'améliorant l'art antérieur des dispositifs métalliques, ne résout pas entièrement le problème que va résoudre la présente invention dont les moyens et fonctions mis en place améliorent très sensiblement les fonctionnalités.

C'est ce que se propose de faire la présente invention par la mise en place de moyens nouveaux nécessaires pour obtenir les résultats attendus.

Les figures servant à la compréhension de l'invention sont :
Figure 1 planche 1/5 vue en coupe du dispositif correspondant au brevet FR 2771280 (Art antérieur : moyens entièrement mécaniques) Figure 2 planche 1/5 vue en coupe du document n° EP 0669109
Figure 3 planche 1/5 vue en coupe du dispositif le plus proche dispositif de la demande PCT WO/03007828 (Art antérieur)

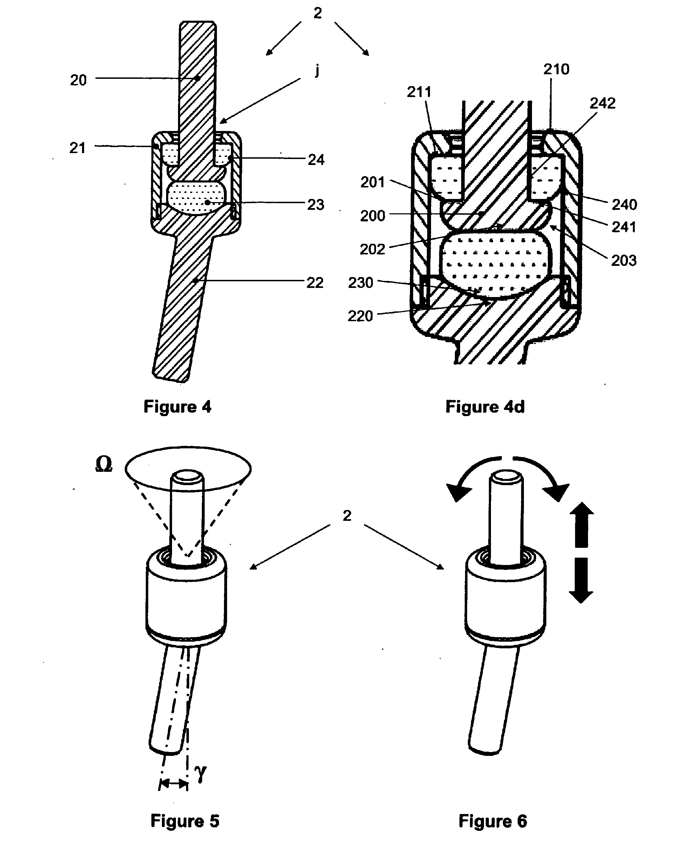

Figure 4 planche 2/5, vue en coupe du nouveau dispositif avec ses moyens mis en oeuvre
Figure 4d planche 2/5, vue de détail du nouveau dispositif avec ses moyens mis en oeuvre
Figure 5 planche 2/5 vue en perspective du nouveau dispositif illustrant ses possibilités de multiaxialité
Figure 6 planche 2/5 vue en perspective du nouveau dispositif travaillant en mode de flexion et traction-compression
Figure 7 planche 3/5 vue en perspective du nouveau dispositif monté sur des vis pédiculaires
Figure 8 planche 3/5 vue arrière du nouveau dispositif monté sur des vis pédiculaires avec le repère de positionnement
Figure 9 planche 3/5 vue arrière du nouveau dispositif monté sur des vis pédiculaires montrant la limite de position basse en compression
Figure 10 et 11 planche 4/5 vue en coupe du nouveau dispositif comportant une butée de sécurité, figure 10 dispositif au repos et figure 11 dispositif en compression
Figure 12 planche 4/5 vue d'un nouveau dispositif monté sur trois vis pédiculaires fixées sur des corps adjacents

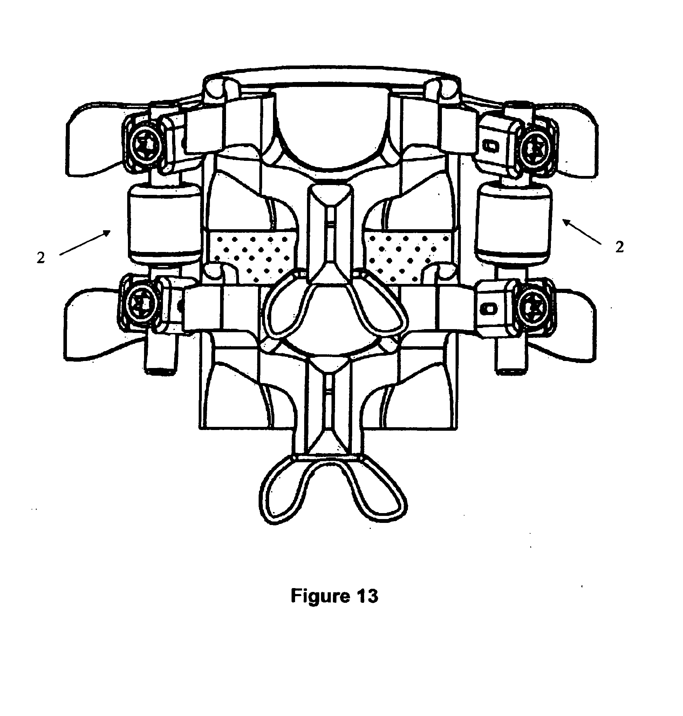

Figure 13 planche 5/5 vue de deux dispositifs objet de l'invention montés sur le rachis

### Dans la réalisation de l'art antérieur la plus proche

Le dispositif (1) le plus proche du nouveau dispositif (2) objet de l'invention comprend :

Un ensemble de moyens rigides combinés avec des moyens viscoélastiques dont les premiers transmettent les efforts et les seconds amortissent les sollicitations dynamiques.

Les moyens rigides sont constitués de trois éléments :
- une tige piston placée dans la partie supérieure
- une tige fixe dans la partie inférieure
- un capot enfermant l'ensemble des moyens viscoélastiques

Les moyens viscoélastiques biocompatibles de préférence en silicone ou en polyuréthane sont constitués :
- d'un cylindre plein viscoélastique tronqué obliquement
- d'un anneau viscoélastique tronqué obliquement dont les faces viennent en contact avec le piston de la tige piston.

Le dispositif 1 est capable de fonctionner avec les tiges et dont l'une, la tige piston, se déplace de manière multiaxiale avec un petit angle de déplacement et des débattements déterminés.

Il existe un orifice placé à l'une des extrémités du capot qui permet un débattement multi axial de la tige piston du dispositif 1.

La forme de cet orifice est définie en fonction des débattements prévus. La tige piston peut ainsi travailler en traction, compression avec un angle α prédéterminé, la tige piston formant alors avec la tige fixe un angle donné dépendant de la forme de l'orifice.

Cette antériorité présente des inconvénients trop importants pour résoudre entièrement le problème posé.

D'une part, la section la plus faible de l'anneau tronqué obliquement subit trop de contraintes sous l'action des sollicitations mécaniques et par conséquent s'altère rapidement.

D'autre part, la tige piston qui forme un angle α avec la tige fixe dans la position neutre vient en butée contre le capot métallique lors de sollicitations importantes ce qui limite les débattements angulaires et donc la mobilité du patient. La section de l'anneau tronqué obliquement présente des zones à parois de faibles épaisseurs induisant le fait que l'anneau oblique ne peut en aucun cas s'opposer aux déplacements de la tige piston vers le bord du capot. Ceci génère des débris d'usure, qui vont aller en s'accentuant et qui sont très préjudiciables à la longévité du dispositif , donc à la santé du patient. On notera également l'absence de force de rappel qui rend préjudiciable la réponse dynamique du dispositif 1 inadapté à la biomécanique du rachis. C'est pourquoi, cette antériorité ne peut pas être utilisée pour résoudre entièrement notre problème.

### Dans une réalisation préférentielle de l'invention :

Le dispositif 2 est une combinaison de moyens rigides et de moyens viscoélastiques permettant au dispositif 2 de former un angle γ en position neutre entre ses deux éléments de fixation aux vis pédiculaires et d'exercer un effort de rappel élastique permanent autour de cette position en amortissant les efforts de compression et de flexion appliqués sur les vis pédiculaires.

Le dispositif 2 comprend :
- Une tige piston 20 mobile comportant une tête de piston 200 à son extrémité
- Un bloc amortisseur viscoélastique 23 comportant une face convexe 230
- Une tige fixe 22 faisant un angle γ par rapport à l'axe du capot 21 qui comporte à l'extrémité située à l'intérieur du dispositif 2, une surface concave 220 qui épouse parfaitement la face convexe 230 du bloc amortisseur viscoélastique 23
- Un anneau viscoélastique 24 logé à l'intérieur du capot 21 renfermant les moyens viscoélastiques 23 et 24.

Les jeux d'assemblage de cet anneau vis à vis de la tige piston 20 et du capot 21 ont été optimisés à partir d'essais en endurance pour conserver un rappel élastique satisfaisant.

Le capot 21 comporte dans sa partie supérieure un orifice conique 210 dont la conicité permet les débattements angulaires de la tige piston mobile 20 pouvant servir également de butée sans exercer de concentration de contrainte tout en limitant les débattements angulaires de la tige. La face intérieure du cône 211 permet également d'offrir la plus large surface de contact possible à l'anneau viscoélastique 24 qui vient en parfait appui sur cette face du capot et limitant ainsi les contraintes dans l'anneau 24.

Le dispositif 2 est fixé aux vis pédiculaires par les extrémités des tiges 20 et 22. Ces deux tiges forment entre elles un angle γ, position neutre du dispositif non contraint. Cet angle γ reproduit l'angle de la lordose anatomique du rachis , position de parfait équilibre, autour de laquelle le dispositif 2 va toujours tendre à revenir grâce à une force de rappel élastique exercée par l'anneau et le bloc viscoélastiques 24 et 23, ceci, quelles que soient les sollicitations dynamiques et les déplacements qui sont imposés à la tige 20.

Le dispositif 2 comprend la tige fixe 22 qui forme l'angle γ par rapport au capot 21 et à la position neutre de la tige piston 20 mobile. La tige piston 20 est positionnée dans l'axe du capot et se déplace dans les deux sens selon cet axe en comprimant le moyen viscoélastique 23 grâce à la face inférieure 202 de la tête de piston 200 lors des mouvements de compression et flexion et en comprimant aussi l'anneau viscoélastique 24 grâce à la face supérieure du piston 201 lors des mouvements de traction et flexion.

Le déplacement axial de la tige piston 20 mobile peut être combiné avec des déplacements angulaires Ω de cette même tige grâce au contact entre les deux faces 201 et 202 du piston 200.

Lorsque le dispositif est au repos, l'absence de jeu induit des contacts sans contraintes entre la tête de piston 200 et les moyens viscoélastiques 23 et 24 grâce au type d'assemblage effectué. La déformation des moyens viscoélastiques 23 et 24 permet une prise d'angle de la tête de piston 200 autour de sa position neutre en fonction des contraintes appliquées. Sous sollicitations mécaniques, les moyens viscoélastiques 23 et 24 sont alors comprimés sur une partie de leur surface et exercent un effort de rappel élastique sur la tête de piston 200 tendant à ramener la tige 20 dans l'axe du capot 21.

Le piston 200 de la tige 20 possède un congé de raccordement 203 sur sa face inférieure 202 qui évite de créer des amorces de rupture du bloc amortisseur viscoélastique 23 lors d'une prise d'angle du piston 200.

De même, l'anneau viscoélastique 24 dispose d'un congé de raccordement 240 sur sa face 241 pour limiter les risques d'amorce de rupture lors d'une prise d'angle du piston 200.

Cet anneau viscoélastique 24 permet également d'exercer un effort de rappel élastique sur les débattements angulaires de la tige 20 par la déformation possible de son diamètre interne 242. Les déformations possibles de l'anneau viscoélastique 24 permettent l'amortissement des débattements angulaires Ω de la tige 20.

La géométrie de l'orifice conique 210 du capot 21 permet de limiter les débattements de la tiges 20 à l'intérieur d'un cône défini par un angle de polyaxialité Ω de l'ordre de 15°.

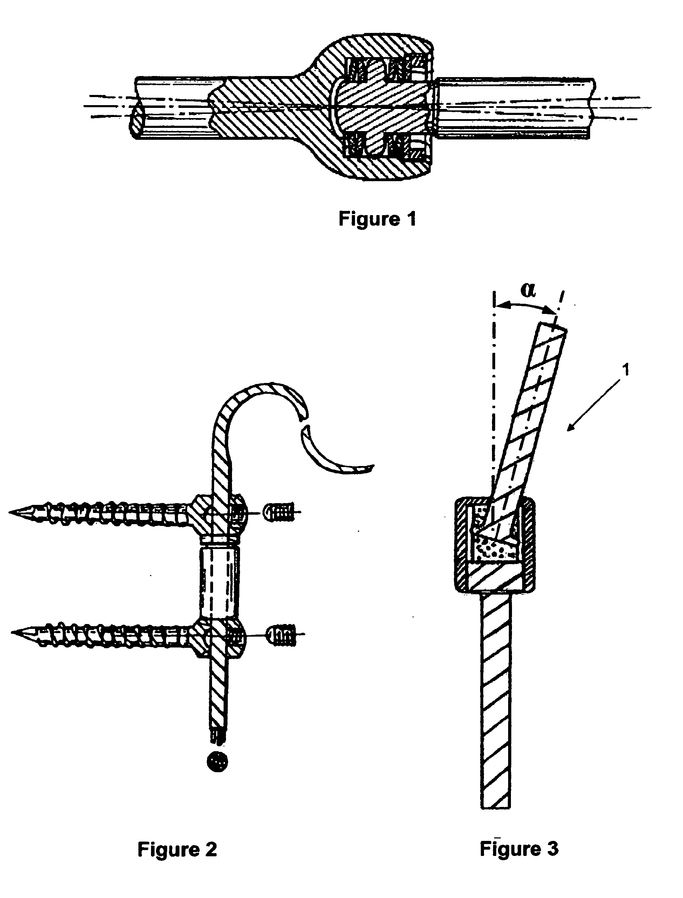

En limite de course, la tige piston 20 mobile vient en appui linéaire sur la surface conique 210 et non en appui ponctuel comme c'était le cas dans l'invention la plus proche de l'art antérieur (figure 3) ce qui à pour effet de réduire les concentrations de contraintes sur la tige piston 20.

Quand le dispositif 2 est soumis à des contraintes de compression, la face inférieure 202 du piston 200 va comprimer l'élément viscoélastique 23, les deux faces en contact étant planes afin de répartir les contraintes sur la totalité des surfaces en contact. La tige fixe 22 comporte à l'extrémité située à l'intérieur du dispositif 2, la surface concave 220 qui épouse parfaitement la face convexe 230 du bloc amortisseur viscoélastique 23.

La concentricité de ces faces convexe et concave qui s'appairent parfaitement, permet le centrage de l'élément viscoélastique 23 avec la tige 22 et le capot 21 de manière à ce que le moyen viscoélastique 23 soit parfaitement centré à l'intérieur du capot 21.

Cette conception permet à l'élément viscoélastique 23 de se déformer sans aller au contact des parois internes du capot 21 tout en évitant les frottements et des sur contraintes très préjudiciables à la longévité du moyen viscoélastique 23.

D'autre part, ce centrage par la concavité 220 et la convexité 230 de la tige fixe 22 et de l'élément viscoélastique 23 empêche tout déplacement radial de l'élément viscoélastique 23 qui pourrait être provoqué par un mouvement en flexion de la tige piston 20, le piston 200 exerçant une pression oblique sur le moyen viscoélastique 23.

C'est pourquoi, le fonctionnement du dispositif 2 est nettement amélioré par rapport au dispositif 1 plus limité dans ses débattements et dans sa durée de vie.

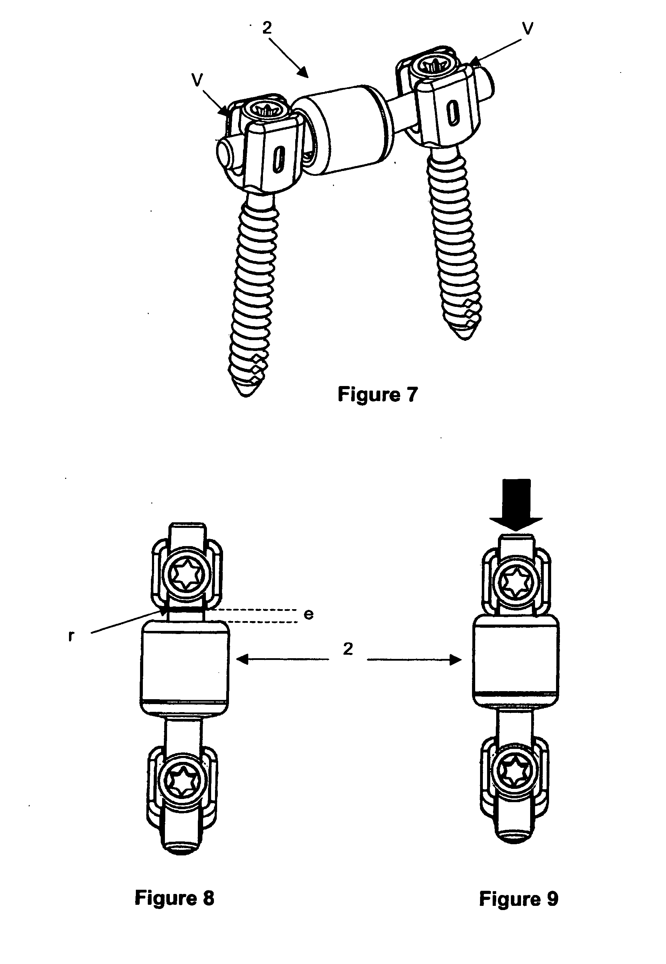

La tige piston mobile 20 comporte un repère r (figures 8 et 9) destiné à une parfaite mise en place du dispositif 2. Ce repère r est visé par l'opérateur lors de la mise en place de la tige piston 20 et doit être aligné avec la face inférieure des têtes de vis pédiculaire V. Cet alignement permet de conserver une distance e (de quelques mm) entre la face supérieure du capot 21 et la face inférieure des têtes de vis pédiculaire V, ceci afin de limiter les contraintes et déplacements induits au delà de cette limite dans l'élément viscoélastique 23.

Dans le cas où on se retrouverait en présence de sollicitations dynamiques trop élevées, la face inférieure de la vis pédiculaire viendrait en butée sur la face supérieure du capot 21 pour limiter les efforts appliqués sur les moyens viscoélastiques du dispositif 2. Ceci garantit une sécurité pour une bonne tenue mécanique en fatigue du dispositif 2 objet de l'invention.

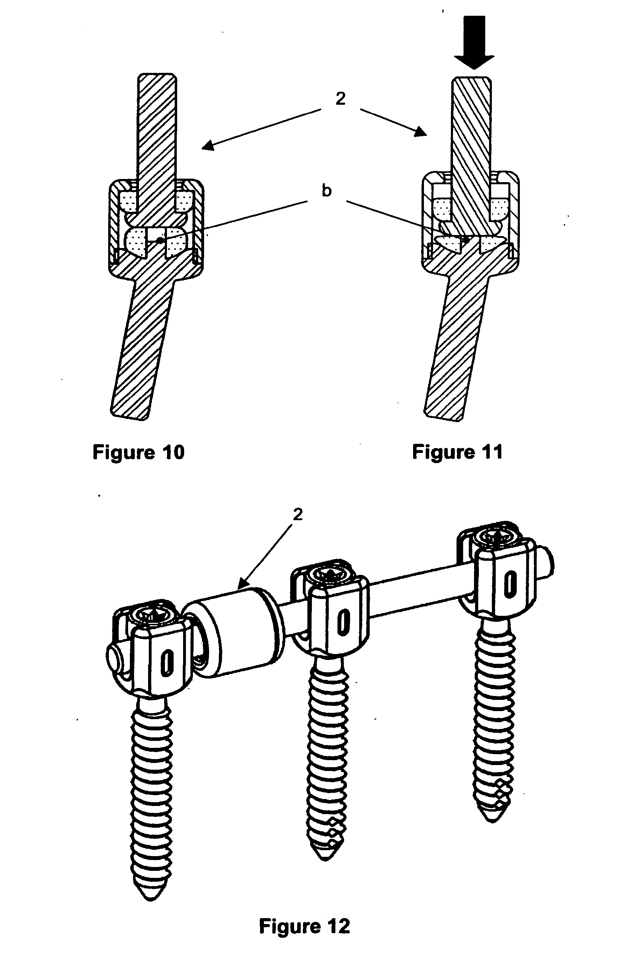

La figure 12 montre comme dans l'antériorité la possibilité d'une combinaison de deux étages vertébraux dont l'un a été fusionné et l'autre rendu mobile par le dispositif 2.

Dans une variante possible appliquée à ce dispositif, on intègre une sécurité supplémentaire (figures 10 et 11) qui est une butée centrale placée au fond de la cavité concave 220 de la tige fixe 22. Lors des efforts de compression, le piston 200 de la tige piston 20 vient en contact avec la butée b après un déplacement vertical prédéfini de l'ordre de quelques millimètres, ce qui évite les surcharges qui pourraient être appliquées sur le moyen viscoélastique 23.

Cette butée b supplémentaire peut se suffire à elle-même ou compléter celle résultant de l'alignement de l'extrémité de la vis avec le repère r.

## Revendications

1. Dispositif (2) de stabilisation postérieur dynamique destiné à être fixé par des vis pédiculaires à un segment intervertébral, comprenant :
- un piston mobile (20),
- une tige fixe (22),
- un capot (21) dans lequel est logée une combinaison de moyens rigides et de moyens viscoélastiques constituée :
- d'une tête de piston (200) placée à une extrémité de la tige piston (20)
- d'un bloc amortisseur viscoélastique (23)
- d'une extrémité de la tige fixe (22)
- d'un anneau viscoélastique (24) logé sans précontrainte avec des jeux optimisés à l'intérieur du capot (21) renfermant les moyens viscoélastiques (23 et 24),
ces moyens permettant au dispositif d'exercer un effort de rappel élastique permanent autour de cette position en amortissant les efforts de compression et de flexion appliqués sur les vis pédiculaires;
***caractérisé* en ce que** :
- le bloc amortisseur viscoélastique (23) possède une face convexe (230),
- la tige fixe (22) fait un angle (γ) par rapport à l'axe du capot (21), ce moyen permettant au dispositif (2) d'épouser la lordose anatomique en position neutre entre les deux éléments de fixation aux vis pédiculaires, la tige fixe (22) comporte à son extrémité une surface concave (220) qui épouse parfaitement la face convexe (230) du bloc amortisseur viscoélastique (23).

2. Dispositif (2) de stabilisation postérieur dynamique selon la revendication 1 **caractérisé en ce que** le capot (21) présente un orifice (210) dont la conicité permet à la tige (20) :
- de limiter ses débattements à l'intérieur de l'orifice conique (210) défini par un angle de polyaxialité (Ω) de l'ordre de 15°,
- et de venir sur un appui non ponctuel linéaire sur la surface conique (210) ce qui réduit les concentrations de contrainte sur la tige piston (20).

3. Dispositif (2) de stabilisation postérieur dynamique selon la revendication 1 **caractérisé en ce que** l'anneau viscoélastique (24) dispose d'un congé de raccordement (240) sur sa face (241) limitant les risques d'amorce de rupture lors d'une prise d'angle du piston (200), le dit anneau viscoélastique (24) permettant également d'exercer un effort de rappel élastique sur les débattements angulaires de la tige (20) par déformation de son diamètre interne (242).

4. Dispositif (2) de stabilisation postérieur dynamique selon la revendication 1 **caractérisé en ce que** la tige piston mobile (20) comporte un repère (r) destiné à une parfaite mise en place du dispositif (2), la visée de ce repère r par l'opérateur effectuée lors de la mise en place de la tige piston (20) impose un alignement avec la face inférieure des têtes de vis pédiculaire (V), cet alignement permettant de conserver une distance (e) entre la face supérieure du capot (21) et la face inférieure des têtes de vis pédiculaire (V), en limitant ainsi les déplacements induits dans l'élément viscoélastique (23) à la distance maximum (e).

5. Dispositif (2) de stabilisation postérieur dynamique selon la revendication 1 **caractérisé en ce qu'**il comprend une butée (b) placée au centre de la cavité concave (220) de la tige fixe (22) qui lors des efforts de compression, amène le piston (200) de la tige piston (20) à venir en contact avec cette butée (b) après un déplacement vertical prédéfini de quelques millimètres, qui évite de surcharger le moyen viscoélastique (23).

6. Dispositif (2) de stabilisation postérieur dynamique selon la revendication 1 **caractérisé en ce que** les faces convexe (230) de l'élément viscoélastique (23) et concave (220) de la tige fixe (22) sont concentriques et s'appairent, ce qui permet le centrage avec le capot (21) de manière à ce que le moyen viscoélastique (23) soit parfaitement centré à l'intérieur du capot (21), permettant à l'élément viscoélastique (23) de se déformer sans aller au contact des parois internes du capot (21) tout en évitant les frottements et des sur-contraintes très préjudiciables à la longévité du moyen viscoélastique (23).

## Patentansprüche

1. Dynamische, hintere Stabilisierungsvorrichtung (2), die für die Befestigung mit Pedikelschrauben an einem intervertebralen Segment vorgesehen ist und Folgendes umfasst:
- einen beweglichen Kolben (20),
- eine feste Stange (22),
- eine Abdeckung (21), die eine Kombination bestehend aus starren und viskoelastischen Mitteln enthält:
- einen Kolbenkopf (200) an einem Ende der Kolbenstange (20)
- einem viskoelastischen Dämpfungsblock (23)
- einem festen Stangenende (22)
- einem viskoelastischen Ring (24)
**dadurch *gekennzeichnet,* dass**:
- der viskoelastische Dämpfungsblock (23) eine konvexe Fläche (230) besitzt,
- die feste Stange (22) einen Winkel (γ) zur Achse einer Abdeckung (21) hat und am Ende eine konkave Fläche (220) besitzt, die perfekt zur konvexen Fläche (230) des viskoelastischen Dämpfungsblocks (23) passt,
- der viskoelastische Ring (24) ohne Vorspannung mit optimiertem Spiel in der Abdeckung (21) sitzt und die viskoelastischen Mittel (23 und 24) umschließt,
mit diesen Mitteln kann die Vorrichtung (2):
- die anatomische Lordose in neutraler Stellung zwischen den zwei Befestigungselementen an den Pedikelschrauben anpassen,
- eine permanente elastische Rückziehung um diese Position ausüben, indem sie die Druck- und Biegebelastung auf die Pedikelschrauben dämpft.

2. Dynamische, hintere Stabilisierungsvorrichtung (2) gemäß Anspruch 1, **dadurch gekennzeichnet, dass** die Abdeckung (21) eine Öffnung aufweist (210), durch deren Konizität die Stange (20) in der Lage ist:
- ihre Ausschläge in der konischen Öffnung (210) zu begrenzen, die durch einen Polyaxialwinkel (Ω) von 15° festgelegt sind,
- und auf der konischen Fläche (210) nicht punktförmig und nicht linear zur Anlage zu kommen, wodurch sich die Spannungskonzentrationen auf die Kolbenstange (20) verringern.

3. Dynamische, hintere Stabilisierungsvorrichtung (2) gemäß Anspruch 1, **dadurch gekennzeichnet, dass** der viskoelastischen Ring (24) eine Ausrundung (240) auf seiner Oberfläche (241) besitzt, die die Bruchgefahr bei einer Schräglage des Kolbens (200) begrenzt, wobei der viskoelastische Ring (24) ebenfalls durch Verformung seines Innendurchmessers (242) eine elastische Rückzugskraft auf die Winkelausschläge der Stange (20) ausübt.

4. Dynamische, hintere Stabilisierungsvorrichtung (2) gemäß Anspruch 1, **dadurch gekennzeichnet, dass** die bewegliche Kolbenstange (20) eine Kennzeichnung (r) besitzt, die das perfekte Einsetzen der Vorrichtung (2) ermöglicht, die Ausrichtung dieser Kennzeichnung r durch den Operateur beim Einsetzen der Kolbenstange (20) verlangt eine Ausrichtung mit der unteren Fläche der Pedikelschraubenköpfe (V), da diese Ausrichtung die Einhaltung eines Abstandes (e) zwischen der oberen Fläche der Abdeckung (21) und der unteren Fläche der Pedikelschraubenköpfe (V) ermöglicht, so dass dadurch die Verschiebung im viskoelastischen Element (23) auf den maximalen Abstand (e) begrenzt werden.

5. Dynamische, hintere Stabilisierungsvorrichtung (2) gemäß Anspruch 1, **dadurch gekennzeichnet, dass** sie einen Anschlag (b) im Zentrum der konkaven Vertiefung (220) der festen Stange (22) besitzt, die den Kolben (200) der Kolbenstange (20) bei Druckbeanspruchung mit diesem Anschlag (b) nach einer vorbestimmten vertikalen Verschiebung von einigen Millimetern in Kontakt bringt, so dass eine Überbelastung des viskoelastischen Mittels (23) vermieden wird.

6. Dynamische, hintere Stabilisierungsvorrichtung (2) gemäß Anspruch 1, **dadurch gekennzeichnet, dass** die konvexen Flächen (230) des viskoelastischen (23) und konkaven (220) Elements der festen Stange (22) konzentrisch und paarweise angeordnet sind; das ermöglicht die Zentrierung mit der Abdeckung (21), so dass das viskoelastische Mittel (23) in der Abdeckung (21) richtig zentriert ist, wodurch sich das viskoelastische (23) Element verformen kann, ohne dass es mit den Innenwänden der Abdeckung (21) in Berührung kommt, und so Reibungen und Überbelastungen vermieden werden, die für die Lebensdauer des viskoelastischen Mittels (23) sehr schädlich sind.

## Claims

1. Posterior dynamic stabilization system (2) to be anchored to an intervertebral segment with pedicle screws, comprising:
- a mobile piston (20),
- a fixed rod (22),
- a casing (21) which houses a combination of rigid and visco-elastic means, comprising:
- a piston head (200) placed at one end of the piston rod (20)
- a visco-elastic shock-absorbing unit (23)
- one end of the fixed rod (22)
- a visco-elastic ring (24)
***characterized* by** the following properties:
- the visco-elastic shock-absorbing unit (23) has a convex surface (230),
- the fixed rod (22) forms an angle (γ) with the axis of a casing (21) and, at its end, has a concave surface (220) which fits perfectly with the convex surface (230) of the visco-elastic shock-absorbing unit (23),
- the visco-elastic ring (24) fits without preload and with optimized clearance inside the casing (21) enclosing the visco-elastic means (23 and 24),
these means allow the system (2):
- to match the contour of the anatomical lordosis in neutral position between the two attachments to the pedicle screws,
- to apply a permanent elastic-return force around this position by absorbing the compression and bending forces applied to the pedicle screws.

2. Posterior dynamic stabilization system (2) as per claim 1 **characterized by** the casing (21) having a tapered hole in it (210) which allows the rod (20):
- to limit its displacement in the tapered hole (210) defined by an angle of polyaxiality (Ω) of 15°,
- and to rest against a linear (non-point) support section on the conical surface (210) which reduces concentrated stress on the piston rod (20).

3. Posterior dynamic stabilization system (2) as per claim 1 **characterized by** the visco-elastic ring (24) having a fillet (240) on its surface (241) which limits the risks of incipient cracks when the piston (200) leans; this visco-elastic ring (24) also applies an elastic-return force on the rod's angular displacement (20) thanks to distortion of its inner diameter (242).

4. Posterior dynamic stabilization system (2) as per claim 1 **characterized by** the mobile piston rod (20) having a reference point (r) ensuring perfect placement of the system (2). If the operator aims for this reference point (r) when installing the piston rod (20), this ensures alignment with the lower surface of the heads of the pedicle screws (V); this alignment allows a distance (e) to be maintained between the top surface of the casing (21) and the lower surface of the heads of the pedicle screws (V), thereby limiting displacement induced in the visco-elastic component (23) at the maximum distance (e).

5. Posterior dynamic stabilization system (2) as per claim 1 **characterized by** having a stop (b) placed at the center of the *concave cavity (220)* in the fixed rod (22), which under compression, brings the piston (200) in the piston rod (20) into contact with the stop (b) after a predefined vertical movement of several millimeters, thereby avoiding overload on the visco-elastic means (23).

6. Posterior dynamic stabilization system (2) as per claim 1 **characterized by** having concentric and matching convex surfaces (230) of the visco-elastic component (23) and concave surface (220) of the fixed rod (22), which ensures centering with the casing (21) so that the visco-elastic mean (23) is perfectly centered inside the casing (21), allowing the visco-elastic component (23) to bend without touching the inner walls of the casing (21) while avoiding friction and overloading which greatly reduce the durability of the visco-elastic means (23).
